# EUROPEAN PATENT APPLICATION

(11) **EP 4 625 127 A1**
(43) Date of publication of application: **01.10.2025**
(21) Application number: 24165800.4
(22) Date of filing: 25.03.2024
(51) Int. Cl.: G06F 3/0484, G06F 3/0487, G06F 3/16, G16H 40/67

(54) **INTERCOM PANNEL INTERFACE**

(71) Applicant: Koninklijke Philips N.V., 5656 AG Eindhoven (NL)
(72) Inventor: AMTHOR, Thomas Erik, Eindhoven (NL); WANG, Xinyu, Eindhoven (NL); SHIRODKAR, Rohit Ramesh, 5656AG Eindhoven (NL)
(74) Representative: Philips Intellectual Property & Standards

(57) **Abstract**

Disclosed herein is an intercom panel interface (108) for mounting to an intercom panel (100) of a medical imaging system (302). The intercom panel comprises: a push-to-talk (PPT) button (102), an intercom panel microphone (104), and an intercom panel speaker (106). The intercom panel interface comprises: a panel hardware interface (110) for communicating with a local computational system (362). The panel hardware interface is for receiving a control command and a remote operator audio stream (384). The panel hardware interface is configured to transmit a subject audio stream (390). The intercom panel interface comprises: a robotic button pusher (112) for actuating the push-to-talk button; an interface microphone (116) configured for receiving subject audio from the intercom panel speaker; an interface speaker (118) configured for providing remote operator audio to the intercom panel microphone; and an audio interface (114) configured for digitizing the subject and rendering the remote operator audio stream.

## Description

### FIELD OF THE INVENTION

The invention relates to medical imaging, in particular to communication between a subject and a remote operator.

### BACKGROUND OF THE INVENTION

In medical imaging modalities such as magnetic resonance imaging (MRI), positron emission tomography (PET), single photon emission tomography (SPECT), and computed tomography (CT) the operator of the medical imaging system is able to communicate with the subject being imaged using an intercom system. A common arrangement is that the operator has an intercom panel for controlling the intercom system. The intercom panel typically has a speaker for listening to an audio signal from the subject, a microphone to pick up the operator's voice and a push-to-talk button. When the push-to-talk button is not engaged, the operator is able to hear audio from the subject. When the push-to-talk button is engaged, the subject is able to hear audio from the operator. Communication using such an intercom system is unidirectional.

United States patent application publication US 2023/0185524A1 discloses a communication system between an imaging bay containing a medical imaging device and a control room containing a controller for controlling the medical imaging device includes an intercom with a bay audio speaker and bay microphone in the imaging bay, and a communication path via which bay audio from the bay microphone is transmitted to the control room and via which instructions are transmitted from the control room to the bay audio speaker. An electronic processing device operatively connected with the communication path is programmed to at least one of (i) generate the instructions; (ii) modify the bay audio and output the modified bay audio in the control room; and/or (iii) analyze the bay audio to determine actionable information, determine a modification of or addition to a medical workflow based on the actionable information, and automatically implement the modification of or addition to the medical workflow.

### SUMMARY OF THE INVENTION

The invention provides for an intercom panel, a medical system, a method and a computer program in the independent claims. Embodiments are given in the dependent claims.

In one aspect an intercom panel interface is disclosed. The intercom panel interface is configured for mounting to an intercom panel of a medical imaging system. The intercom panel comprises a push-to-talk button, an intercom panel microphone, and an intercom panel speaker. The intercom panel interface further comprises a panel hardware interface configured for communicating with a local computational system. The panel hardware interface is configured for receiving a control command from the local computational system. The panel hardware interface is configured for receiving a remote operator audio stream from the local computational system. The panel hardware interface is further configured to transmit a subject audio stream to the local computational system. The intercom panel interface further comprises a robotic button pusher configured to actuate the push-to-talk button in response to receiving the control command. The intercom panel interface further comprises an interface microphone configured for receiving subject audio from the intercom panel speaker. The intercom panel interface further comprises an interface speaker configured for providing remote operator audio to the intercom panel microphone. The intercom panel interface further comprises an audio interface configured for digitizing the subject audio into the subject audio stream. The audio interface is further configured for rendering the remote operator audio stream to the interface speaker.

In a further aspect a medical system is disclosed. The medical system comprises an example of the intercom panel interface. The medical system further comprises a local memory storing local machine-executable instructions and a speech detection module configured for detecting human speech in an input digital audio stream. The medical system further comprises a local network interface configured to form a network connection with the remote operator station. The medical system further comprises the local computational system. Execution of the local machine-executable instructions causes the local computational system to e.g., continually receive the remote operator audio stream via the network interface. The execution of the local machine-executable instructions further causes the local computational system to continually detect the human speech within the remote operator audio stream by inputting the remote operator stream into the speech detection module as the input digital audio stream. Execution of the local machine-executable instructions further causes the local computational system to continually provide the control signal and the remote operator audio stream to the panel hardware interface when the human speech is detected. Execution of the local machine-executable instructions further causes the local computational system to continually transmit the subject audio stream to the remote operator station.

This aspect may be beneficial because it may provide for a means of seamlessly controlling the intercom panel of a medical imaging system remotely. The intercom panel interface provides a means simultaneously providing a connection to the intercom panel interface and the intercom panel interface as well as providing mechanical control of the push-to-talk button.

In another aspect, a method of operating a medical system is disclosed. The method comprises receiving the remote operator audio stream via the network interface. The method further comprises detecting the human speech within the remote operator audio stream by inputting the remote operator stream into the speech detection module as the input digital audio stream. The method further comprises providing the control signal and the remote operator audio stream to the panel hardware interface when the human speech is detected. The method further comprises transmitting the subject audio stream to the remote operator station.

This example may be beneficial because it may enable more complicated means of controlling the flow of audio information to and from the intercom panel. For example, if there were a local operator sitting in front of the intercom panel interface and then pushed the push-to-talk button on the intercom panel then this would disable the audio coming from the subject in the medical imaging system. More complicated logic could be used to determine when the control signal is able to actuate the push-to-talk button using the robotic button pusher.

In another aspect, a computer program that comprises machine-executable instructions and is configured for controlling a local computational system to perform an example of the method is disclosed.

### BRIEF DESCRIPTION OF THE DRAWINGS

In the following preferred embodiments of the invention will be described, by way of example only, and with reference to the drawings in which:
Fig. 1 illustrates an example of an intercom panel and an intercom panel interface.
Fig. 2 illustrates a portion of an intercom panel interface where the manual actuation of the push-to-talk button is prevented.
Fig. 3 illustrates an example of a medical system.
Fig. 4 shows a flow chart which illustrates a method of using the medical system of Fig. 1.
Fig. 5 illustrates a functional diagram depicting a further example of a medical system.

### DESCRIPTION OF EMBODIMENTS

Like numbered elements in these figures are either equivalent elements or perform the same function. Elements which have been discussed previously will not necessarily be discussed in later figures if the function is equivalent.

Examples may provide a means of establishing a direct patient communication from a remote operator supporting a local operator. Some examples may provide a medical system that enables a remotely initiated two-way audio communication system with the subject. In some examples the system is further configured for automatically correcting for signal delays, and building on existing patient intercom using a robotic button pusher (for initiating the communication). Some examples may provide a retrofitted set of speaker and microphone, as well as a mechanical button pusher to be connected to the intercom device on the technologist's desk to allow for remotely initiated two-way audio communication with the patient. Examples may further provide a medical system and method to automatically toggle the audio direction at the local intercom based on the detected remote audio signal and buffer the remote expert's voice in such a way that the delay induced by the mechanical button pusher does not cut off the audio signal received from the remote expert.

Due to shortage of radiology staff and the increasing need to improve efficiency and cost-effectiveness of radiologic procedures, remote expert support systems for radiologic imaging, such a Radiology Operations Command Center (ROCC), are gaining importance in clinical practice. Communication of the local operator with the subject in the imaging room is usually realized via an intercom system, allowing the technologist at the control desk to hear and talk to the patient. In many situations, the remote expert supporting the imaging examination, would like to have a way to directly communicate with the patient, for example to identify the patient, ask for details of the disease or anatomy, give instructions for the upcoming MRI scan, etc. This is a highly requested yet unavailable feature for ROCC.

In an example, an intercom panel interface is configured for mounting to an intercom panel of a medical imaging system. The intercom panel comprises a push-to-talk button, an intercom panel microphone, and an intercom panel speaker. When the push-to-talk button is engaged, the intercom panel microphone is able to pick up speech, sound, signals of interest (e.g., alarms), or other noises or sounds, and then play it back for a subject which is currently being imaged in the medical imaging system. When the push-to-talk button is released, the intercom panel speaker provides an audio channel so that the operator may listen to speech or other noises coming from where the subject is in the medical imaging system. In some embodiments, the intercom panel microphone is switched off while the intercom panel is released. The use of a push-to-talk button causes the audio in the intercom system to be transmitted in one direction, e.g., from local operator to remote operator, from local patient to remote operator, from both the local operator and patient to the remote operator, and vice-versa.

The intercom panel interface further comprises a panel hardware interface configured for communicating with a local computational system. The panel hardware interface may take different forms in different embodiments and examples. In one instance, the panel hardware interface may be the hardware interface of a local computational system. In other examples the panel hardware interface may be a network or data communication component such as a Bluetooth connection or a USB connection. The panel hardware interface, at least in some embodiments, is configured for receiving a control command from the local computational system. The panel hardware interface is configured for receiving a remote operator audio stream from the local computational system. The panel hardware interface is further configured to transmit a subject audio stream to the local computational system. The subject audio stream may encompass an audio stream that contains audio from a patient, personnel, or other subjects in or around the imaging bay or console. The panel hardware interface may function as a control component for controlling the various components of the intercom panel interface.

The intercom panel interface further comprises a robotic button pusher that is configured to actuate the push-to-talk button in response to receiving the control command. This enables the actuation of the push-to-talk button remotely. This may for example enable a remote operator located at a site remote to the medical imaging system to control the intercom system. The intercom panel interface further comprises an interface microphone configured for receiving subject audio from the intercom panel speaker. For example, the intercom panel microphone is positioned adjacent or near the intercom panel speaker. The intercom panel interface further comprises an interface speaker that is configured for providing remote operator audio to the intercom panel microphone. For example, the interface speaker may be configured to focus the remote operator audio to the intercom panel microphone. The intercom panel interface further comprises an audio interface configured for digitizing the subject audio into the subject audio stream. The audio interface is further configured for rendering the remote operator audio stream to the interface speaker.

This example may be beneficial because it may provide for a means of seamlessly controlling the intercom panel of a medical imaging system remotely.

In another example, the intercom panel is configured to cover the push-to-talk button of the intercom panel such that the manual actuation of the push-to-talk button is prevented. The intercom panel interface further comprises a manual talk button. The intercom panel interface is further configured such that pushing the manual talk button generates the control command to actuate the push-to-talk button of the intercom panel. This example may be beneficial because it may enable more complicated means of controlling the flow of audio information to and from the intercom panel. For example, if there were a local operator sitting in front of the intercom panel interface and then pushed the push-to-talk button on the intercom panel then this would disable the audio coming from the subject in the medical imaging system. More complicated logic could be used to determine when the control signal is able to actuate the push-to-talk button using the robotic button pusher.

In another example, a medical system is disclosed that comprises an example of the intercom panel interface according to any of the embodiment of the present application. The medical system further comprises a local memory storing machine-executable instructions and a speech detection module that is configured for detecting human speech in an input digital audio stream. The digital audio stream may for example be input into the speech detection module and the speech detection module may identify if there is human speech in this input digital audio stream or not. The speech detection module may be implemented in a variety of ways such as is described below. The medical system further comprises a local network interface that is configured to form a network connection with a remote operator station. The remote operator station may for example be enabled to remotely control the medical imaging system. The medical imaging system could be any one of: PET (Positron Emission Tomography), MRI (Magnetic Resonance Imaging), CT (Computed Tomography), US (Ultrasound), X-ray, PET-CT, SPECT-CT, pathology equipment, Fluoroscopy, Mammography, CBCT (Cone Beam CT), or any other imaging equipment used in the field for diagnosing patients.

A difficulty with controlling medical imaging systems remotely is that the software controls may not be available or the software for operating the medical imaging system may be so old that it is not desirable to modify the software to enable remote control of the device. A means of controlling a medical imaging system remotely may be to clone the screen of the computer system controlling the medical imaging system such as using a screen duplicator and to provide interfaces such as a keyboard and mouse interface which works over the internet or other network connection. A remote operator can then control the medical imaging system as if the operator were in the control room of the medical imaging system. The use of the intercom panel interface, as described above, may enable the remote operator to operate and control the intercom system remotely. This for example may enable the remote operator to control the medical imaging system as if the operator were in the control room of the medical imaging system.

The medical system further comprises a local computational system. Execution of the local machine-executable instructions causes the local computational system to continually receive the remote operator audio stream via the network interface. Execution of the machine-executable instructions further causes the computational system to detect the human speech within the remote operator audio stream by inputting the remote operator audio stream into the speech detection module as the input digital audio stream. Execution of the machine-executable instructions further causes the computational system to continually provide the control signal and the remote operator audio stream to the panel hardware interface when the human speech is detected. Execution of the machine-executable instructions further causes the computational system to transmit the subject audio stream to the remote operator station. In this example the robotic button pusher is controlled to actuate the push-to-talk button when human speech is detected in the remote operator audio stream. This means that the operator can use the intercom panel of the medical imaging system remotely without the need to push the button. The remote operator could for example use a headset or other audio system such is typically used for teleconferencing. This may enable the integration of a remote operator into the control of the medical imaging system without the need of specialized hardware at the location of the remote operator.

In another example, the speech detection module is configured to detect the human speech by determining when the average audio signal is above a predetermined signal strength for at least a predetermined time. For example, if there is a noise or speech above the predetermined signal strength for at least a predetermined time then the system will determine that this is speech or noise or sound which should be transmitted to the subject remotely.

For example, the predetermined signal strength could refer a signal strength above the background noise level in the room from which the audio signal is captured (i.e. the remote control room or the local exam room). In case of an MR exam room, there is an inherent noise background of the cryogenic cooling system to be considered. In the case of a remote operator control room, there may be background noise from air conditioning or from distant voices. Signal strengths significantly larger, e.g. 50% or 100% larger, than the background noise level would be considered speech. If such a larger signal is identified for, e.g., at least 0.3 seconds, the system may decide that someone is talking.

In another example, the speech detection module is configured to detect the human speech by comparing an FFT (Fast Fourier Transform) of the digital audio stream against a predetermined FFT distribution. In this example, one method would be to observe the signal in the frequency domain and to use simple pattern matching on the frequency spectrum or monitor the signal intensity in a predetermined frequency range. In this way for example, a human voice could be distinguished from an alarm bell or noise which has a different frequency component or spectrum. In one example, the audio signal is observed in the frequency domain and use simple pattern matching on the frequency spectrum or monitor the signal intensity in a predefined frequency range. In this way, for example, a human voice could be distinguished from an alarm bell.

In another example, the speech detection module is configured to detect the human speech using a trained neural network. In this example, a trained neural network may be able to distinguish speech from the background noise more specifically. It may capture the intended conversation and notifies the system to generate the control signal. For example, when a remote radiologist talks to the microphone the trained neural network would classify this as human speech and trigger a message that activates the control signal. If there is a vacuum cleaner or other noise working in the background the trained neural network would classify this as background noise and thus would not trigger the control signal.

The trained Neural Network (NN) for example could be a recurrent neural network (RNN) and trained using various clips of audio that do and do not contain human speech. This could be trained using a standard deep learning algorithm to train the recurrent neural network. An example of an applicable RNN could be LSTM (Long Short-term Memory) or B-RNN (Bidirectional RNN) or a combination of both. Each of these architectures are capable of performing speech recognition. Therefore, either of these RNN architectures may be used for speech detection when implemented in various examples.

In one example, a trained NN is capable of distinguishing human speech from background noise, more specifically, it may capture the intended conversation, and is used to control the intercom panel interface. For example, when a remote radiologist talks on the microphone, the trained NN would classify this as human speech, and trigger a message to the audio and button control module. Whereas, if there is a vacuum cleaner working in the background, the trained NN would classify this as background noise, and thus won't trigger any notification to the audio and button control module. To achieve this task, a selected NN could be first fed with a labelled dataset of various samples of human speech and background noise. Via training, the NN's parameter values would be updated, and its performance would be validated. Then the validated NN would be saved as a file and loaded onto the application. The trained NN could be designed to further updating its parameter values after deployment, based on actual conversations and user feedback.

In another example, execution of the machine-executable instructions further causes the computational system to buffer the remote operator audio stream. The buffer, as used herein, encompasses a memory which is able to store the remote operator audio stream. This may for example be used to delay the remote operator audio stream.

In another example, execution of the machine-executable instructions further causes the computational system to delay transmission of the remote operator audio stream using the buffered remote operator audio stream by a predetermined robot button pusher delay. The predetermined robot button pusher delay, as used herein, may encompass a delay which accounts for a lag in time before the robotic button pusher activates the push-to-talk button. This may include mechanical delays of the robotic button pusher as well as additional time allowed for the transmission of the control signal via a network connection or over the internet.

In another example, execution of the machine-executable instructions further causes the computational system to (continually) detect the human speech within the subject audio stream by inputting the subject audio stream into the speech detection module as the digital audio stream. Execution of the machine-executable instructions further causes the computational system to (continually) delay the transmission of the control command when human speech is detected in the subject audio stream. Execution of the machine-executable instructions further causes the computational system to (continually) delay the transmission of the remote operator audio stream when the subject speech is detected in the subject audio stream using the buffered remote operator audio stream.

This example may be beneficial because the system automatically may prevent the engaging of the push-to-talk button when the subject is speaking. For example, if an operator pushes the push-to-talk button then this would prevent audio from the subject from being heard. If the subject is already speaking, then the speech from the remote operator is delayed. This may for example provide for better communication as well as improved safety when using the medical system.

In another example, execution of the machine-executable instructions further causes the computational system to generate a time-compressed remote operator audio stream from the buffered remote operator audio stream when the buffered remote operator audio stream is delayed with respect to the remote operator audio stream. The time-compressed remote operator audio stream is transmitted to the panel hardware interface as the remote operator audio stream. In this example, when the remote operator audio stream has been delayed such as when the subject is speaking or when there is a delay due to the predetermined robot button pusher delay, the buffered remote operator audio stream is able to catch up to the present time by using the compressed remote operator audio stream. Various commercially available time-compression algorithms may be used to accelerate the compressed remote operator audio stream without changing the pitch of the voice. Using this functionality, the output audio signal can be played in a slightly accelerated speed until the buffer is played out fully and there is no time lag any more between the buffer output and the speech input. Once this situation has been reached, the original in put signal can be passed through directly to the output.

In another example, execution of the machine-executable instructions further causes the computational system to pause the remote operator audio stream using the buffered remote operator audio stream when the push-to-talk button is manually pressed, or when the local operator speech is detected in the subject audio stream. In this example, the speech by the remote operator is paused when a local operator is talking, or the push-to-talk button is manually pressed. This may be advantageous because it may reduce the chance that the various operators talk over each other, and some information is lost.

In another example, the local memory further stores an audio noise filtering module configured for removing audio noise. For example, the audio noise filtering module may remove various spectral components of noise which are normally outside of the normal spectrum of human speech, for example high-pass and low-pass filters may be used. In other examples a neural network trained to remove noise from a speech signal may be used to remove noise which is not speech. Execution of the machine-executable instructions further causes the computational system to remove audio noise from any one of the following using the audio noise filtering module: the remote operator audio stream and the subject audio stream. This may be beneficial because it may help the subject and the remote operator to communicate more effectively.

In another example, the local memory further comprises an alarm identification module that is configured to output an alarm signal in response to detecting an audible alarm in the subject audio stream. Execution of the machine-executable instructions further causes the computational system to detect the audible alarm by inputting the subject audio stream into the alarm identification module and then to provide the alarm signal to the medical system if the audible alarm is detected. For example, a neural network which is used may be trained to identify alarm signals or, for example, an FFT may be taken of the subject audio stream and components which are tailored to the particular audible alarm may enable easy detection of the audible alarm. This example may be beneficial because it may help to improve the safety of the medical system.

In another example, the medical system further comprises the remote operator station. The remote operator station is configured for remote control of the medical imaging system. As was described above, this may be beneficial because the use of the intercom panel may enable easier and better remote control of the medical system.

In another example, the medical system further comprises the medical imaging system. The medical imaging system may for example be a magnetic resonance imaging system, a digital fluoroscope, a computed tomography system, a positron emission tomography system, or a single-photon emission tomography system or any other medical imaging system.

In another example, the medical system further comprises the intercom system. The intercom system comprises the intercom panel.

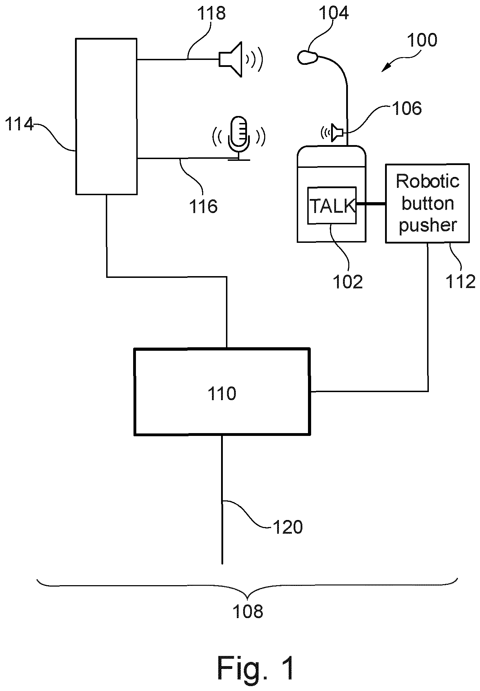

Fig. 1 illustrates an intercom panel 100 and intercom panel interface 108. The intercom panel 100 comprises a push-to-talk button 102, an intercom panel microphone 104, and an intercom panel speaker 106. The intercom panel speaker 106 enables an operator in the vicinity of the intercom panel 100 to hear what a subject is saying when the push-to-talk button 102 is not depressed. When the push-to-talk button 102 is depressed the intercom panel microphone 104 becomes active and the intercom panel speaker 106 is deactivated. The intercom panel microphone 104 is then able to pick up audio noises and speech within the vicinity of the intercom panel microphone 104 and then provide audio to a subject in a medical imaging system. The intercom panel 100 is useful when there is an operator in the same room. The intercom panel interface 108 enables the use of the intercom panel 100 remotely.

When working with older, or legacy medical imaging systems, or medical imaging systems from other vendors, the intercom panel interface 108 may enable use of the intercom panel 100 without modifications to the existing system. The intercom panel interface 108 has a panel hardware interface 110 that is connected to a robotic button pusher 112 and an audio interface 114. When the panel hardware interface 110 receives a control signal, it passes this to the robotic button pusher 112 and this causes the robotic button pusher 112 to remotely depress the push-to-talk button 102. The audio interface 114 is shown as being connected to an interface microphone 116 and an interface speaker 118. The interface microphone 116 is placed in the vicinity of the intercom panel speaker 106 and is able to pick up noise or sound or speech generated by the intercom panel speaker 106. The interface speaker 118 is located in the vicinity of the intercom panel microphone 104 and is able to remotely provide audio to the intercom panel microphone 104. The interface microphone 116 is configured for receiving subject audio from the intercom panel speaker 106. The interface speaker 118 is configured for providing remote operator audio to the intercom panel microphone 104. The audio interface 114 is configured for digitizing the subject audio into the subject audio stream. The audio interface 114 is further configured for rendering the remote operator audio to the interface speaker 118.

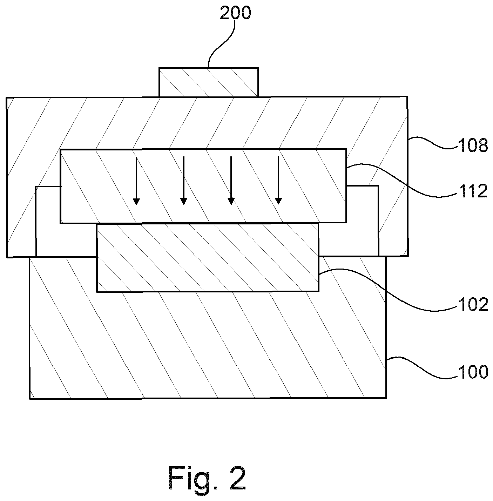

Fig. 2 shows a cross-sectional view of one example of an intercom panel interface 108 mounted to the intercom panel 100. In this example, the intercom panel interface 108 shields the surface of the push-to-talk button 102 such that it cannot be manually depressed. In lieu of this manual actuation there is a manual talk button 200 on the surface of the intercom panel interface 108. When the manual talk button 200 is depressed, this may cause the panel hardware interface 110 to generate a control signal which causes the robotic button pusher 112 to actuate and push the push-to-talk button 102. This may be advantageous because it may enable more complicated means of controlling when the push-to-talk button 102 is depressed. For example, it may prevent a local operator from talking over when the subject is speaking. For example, if speech from the subject is detected or if there is a large amount of noise being generated, then the robotic button pusher 112 may for example be temporarily disabled to enable the subject to finish talking.

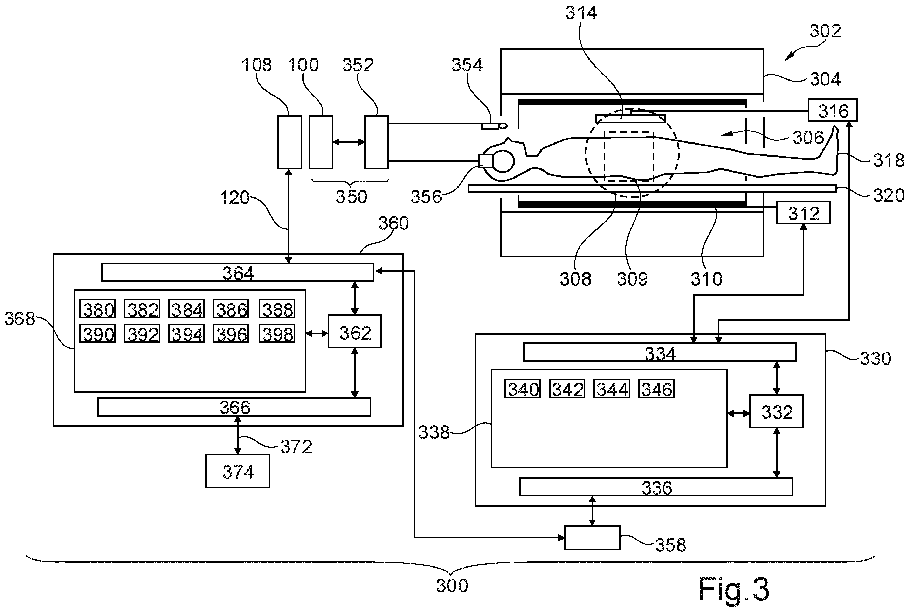

Fig. 3 illustrates an example of a medical system 300. In this example there is a magnetic resonance imaging system 302. The magnetic resonance imaging 302 is exemplary. For example, the magnetic resonance imaging 302 could be replaced with other types of imaging systems or tomographic imaging systems.

The magnetic resonance imaging system 302 comprises a magnet 304. The magnet 304 is a superconducting cylindrical-type magnet with a bore 306 through it. The use of different types of magnets is also possible; for instance, it is also possible to use both a split cylindrical magnet and a so-called open magnet. A split cylindrical magnet is similar to a standard cylindrical magnet, except that the cryostat has been split into two sections to allow access to the iso-plane of the magnet, such magnets may for instance be used in conjunction with charged particle beam therapy. An open magnet has two magnet sections, one above the other with a space in-between that is large enough to receive a subject: the arrangement of the two sections area similar to that of a Helmholtz coil. Open magnets are popular, because the subject is less confined. Inside the cryostat of the cylindrical magnet there is a collection of superconducting coils.

Within the bore 306 of the cylindrical magnet 304 there is an imaging zone 308 where the magnetic field is strong and uniform enough to perform magnetic resonance imaging. A field of view 309 is shown within the imaging zone 308. The k-space data that is acquired typically acquired for the field of view 309. The region of interest could be identical with the field of view 309 or it could be a sub volume of the field of view 309. A subject 318 is shown as being supported by a subject support 320 such that at least a portion of the subject 318 is within the imaging zone 308 and the field of view 309.

Within the bore 306 of the magnet there is also a set of magnetic field gradient coils 310 which is used to spatially encode magnetic spins within the imaging zone 308 of the magnet 304. The magnetic field gradient coils 310 connected to a magnetic field gradient coil power supply 312. The magnetic field gradient coils 310 are intended to be representative. Typically, magnetic field gradient coils 310 contain three separate sets of coils for spatially encoding in three orthogonal spatial directions. A magnetic field gradient power supply supplies current to the magnetic field gradient coils. The current supplied to the magnetic field gradient coils 310 is controlled as a function of time and may be ramped or pulsed.

Adjacent to the imaging zone 308 is a radio-frequency coil 314 for manipulating the orientations of magnetic spins within the imaging zone 308 and for receiving radio transmissions from spins also within the imaging zone 308. The radio frequency antenna may contain multiple coil elements. The radio frequency antenna may also be referred to as a channel or antenna. The radio-frequency coil 314 is connected to a radio frequency transceiver 316. The radio-frequency coil 314 and radio frequency transceiver 316 may be replaced by separate transmit and receive coils and a separate transmitter and receiver. It is understood that the radio-frequency coil 314 and the radio frequency transceiver 316 are representative. The radio-frequency coil 314 is intended to also represent a dedicated transmit antenna and a dedicated receive antenna. Likewise, the transceiver 316 may also represent a separate transmitter and receivers. The radio-frequency coil 314 may also have multiple receive/transmit elements and the radio frequency transceiver 316 may have multiple receive/transmit channels. The transceiver 316 and the gradient controller 312 are shown as being connected to the hardware interface 106 of the computer system 102.

The magnetic field gradient coil power supply 312 and the transceiver 316 are shown as being connected to a control room hardware interface 334 of a control room computer 330. The control room computer 330 may be the computer that a local operator uses to control and operate the magnetic resonance imaging system. The control room computer 330 is shown as having a control room computational system 332 that is in communication with the control room hardware interface 334 as well as a control room user interface 336 and a control room memory 338. The control room memory 338 is intended to represent various types of memory which are accessible to the control room computational system 332. The control room computational system 332 may represent various types of computing cores or computational systems located at one or more locations. The control room hardware interface 334 may provide a user interface which enables an operator to control and operate the magnetic resonance imaging system 302. This may for example provide displays as well as human interface devices such as mice, trackballs, and keyboards.

The control room memory 338 is shown as containing control room machine-executable instructions 340. The control room machine-executable instructions 340 enable the control room computational system 332 to perform such tasks as image processing and the control of the magnetic resonance imaging system 302 components. The memory 338 is further shown as containing pulse sequence commands 342. The pulse sequence commands 342 enable the computational system 332 to control the magnetic resonance imaging system 302 to acquire k-space data 344 according to a magnetic resonance imaging protocol. The memory 338 is shown as containing k-space data 334 that was acquired by controlling the magnetic resonance imaging system 302 with the pulse sequence commands 342. The memory 338 is further shown as containing a magnetic resonance image 346 that was reconstructed from the k-space data 344.

The medical system 300 is further shown as comprising an intercom system 350. The intercom system 350 comprises the intercom panel 100 and a subject interface 352. The subject interface 352 is connected to a subject microphone 354 and subject headphones 356. When the push-to-talk button 102 is not depressed the subject microphone 354 is active and audio is produced by the intercom panel speaker 106. When the push-to-talk button 102 is depressed the intercom panel microphone 104 is active and the subject 318 is able to hear this audio through the subject headphones 356. The push-to-talk button 102 decides which of the microphones 354, 104 is active.

The medical system 300 is further shown as comprising a remote user interface 358. The remote user interface 358 may for example provide a clone or duplicate of the display provided by the control room user interface 336 as well as an emulation of various human interface devices. For example, it may also plug into a USB port and emulate a mouse and a keyboard. The remote user interface 358 therefore enables a remote user to use the control room computer 330 remotely without the installation of additional software on the control room computer.

Adjacent to the intercom panel 100 of the intercom system 350 is the intercom panel interface 108. The intercom panel interface 108 and the intercom panel 100 are arranged as is displayed in Fig. 1. Both the intercom panel interface 108 and the remote user interface 358 are shown as being connected to a local hardware interface 364 of a local computer system 362. In this example, the remote user interface 358 is shown as being operated and controlled by the local computer 360. However, this is not necessarily the case. In some examples, the remote user interface 358 may work independently of the local computer 360.

The local computer 360 is shown as comprising a local computational system 362 that is in communication with the local hardware interface 364, a network interface 366, and a local memory 368. The network interface 366 is shown as forming a network connection 372 with a remote operator station 374. The remote operator station 374 is a computer system used by a remote operator to control the magnetic resonance imaging system 302 remotely. In this example, the connections to the remote user interface 358 are through the local computer 360. In some examples, the remote user interface 358 has a direct connection via an additional network connection to the remote operator station 374.

The local memory 368 is shown as containing local machine-executable instructions 380 that enable the local computational system 362 to perform basic computational tasks and data processing. The local memory 368 is further shown as containing a speech detection module 382 which is able to detect human speech in a digital audio stream. The memory 368 is further shown as containing a remote operator audio stream 384 received from the remote operator station 374. The local memory 368 is further shown as containing a detection of human speech 386 that was determined by inputting the remote operator audio stream 384 into the speech detection module 382. This causes the generation of a control signal 388 which causes the robotic button pusher 112 to activate the push-to-talk button 102. The local memory 368 is further shown as containing a subject audio stream 390 that was received from the intercom panel interface 108.

In this example, the local computer 360 is shown as being a discreet component in comparison to the clone user interface 358 as well as the intercom panel interface 108. In other examples, two or three of these components could have their functionality combined. For example, the panel hardware interface 110 could be identical with the local hardware interface 364.

The memory 368 is also shown as containing a couple of optional software components. There is an optional audio noise filtering module 392 which can be used to remove noise from digital audio streams. There is also an optional alarm identification module 394 which can be used to detected alarms in the subject audio stream 390. There is also a buffered remote operator audio stream 396 that may be generated when the remote operator audio stream 384 is temporarily stopped to enable the subject 318 to speak. The memory 368 is also shown as containing an optional audio time-compression algorithm 398 which can be used to compress the buffered remote operator audio stream 396 to accelerate its playback so that it is able to catch up with the current timeframe.

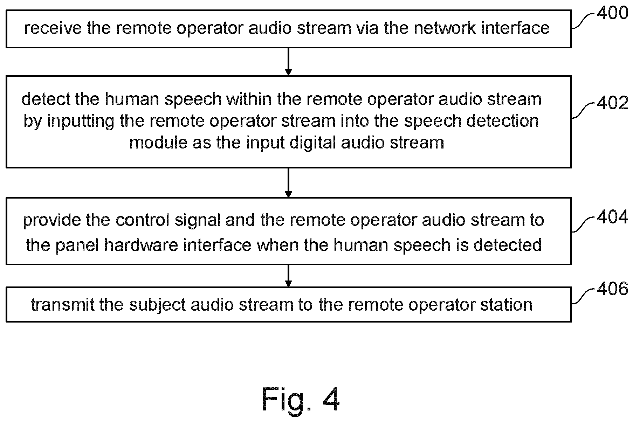

Fig. 4 shows a flowchart which illustrates a method of operating the medical system 300 of Fig. 3. In step 400, the remote operator audio stream 384 is received via the network interface 366. In step 402, the human speech 386 is detected within the remote operator audio stream 384 by inputting the remote operator audio stream 384 into the speech detection module 382 as the input digital audio stream. In step 404, the control signal 388 and the remote operator audio stream 384 are provided to the panel hardware interface 110 when the human speech 386 is detected. In step 406, the subject audio stream 390 is transmitted to the remote operator station 374.

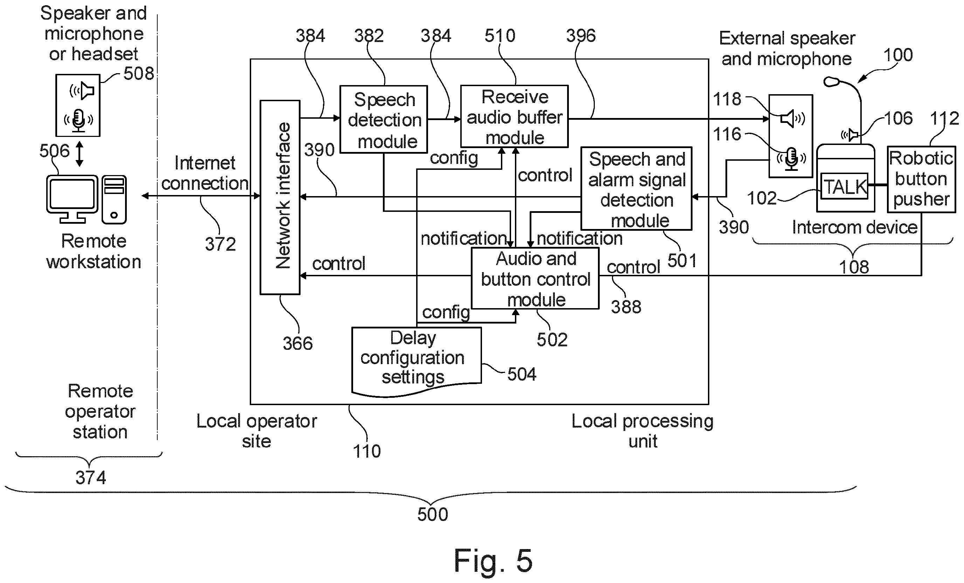

Fig. 5 illustrates a further example of a medical system 500. The medical system is shown as comprising the remote operator station 374 and the panel hardware interface 110. In this example, all of the functionality is implemented using the panel hardware interface 110 instead of the local computational system 362. The functionalities of these components may be combined. The remote operator station 374 comprises a remote workstation 506 that enables a remote operator to provide and listen to audio using a speaker and microphone or a headset 508. The remote workstation 506 is connected via a network connection 372 to the network interface 366 of the panel hardware interface 110. Again, the components and functionality of the local computer system 360 and the panel hardware interface 110 have been combined.

The network interface 366 is able to send and receive the remote operator audio stream and the subject audio stream 390. The remote operator audio stream 384 is fed to the speech detection module 382. The speech detection module 382 may detects speech in the audio signals received from the microphone at the remote workstation, refining the signals by removing background noise, and notifying the `Audio and button control module' 502 about the reception of an upcoming conversation from the remote workstation. The audio and button control module determines 502 the time when the "talk to patient" function becomes active (or the time delay between initiation of final activation of the talk function) either by receiving a corresponding acknowledgement from the robotic button pusher 112, or by being provided with a pre-defined delay configuration setting specific for the type of button pusher and the model of the intercom device.

Upon receiving the message of "remote tech's speech detected" from the Speech detection module 382, The audio and button control module 502 sends command to the Robotic button pusher 112 to enable the `talk to patient' function on of the intercom device 100.

Once the "talk to patient" function is activated, the audio and button control module 502 instructs the receive audio buffer module 510 to start playing the recorded audio buffer with a negative time offset corresponding to the time it took to activate the "talk to patient" function. It may optionally also instruct the receive audio buffer module 510 to accelerate the output audio (without changing pitch) until the delay between input and output audio has decreased to (almost) zero.

The audio and button control module further 510 receives information from the speech and alarm signal detection module (501) (for the patient audio) described below. When patient speech is detected while the system is already in the process of initiating the "talk to patient" function, there is a risk that the remainder of the sentence spoken by the patient is lost once the button is completely pushed. In this case, the audio and button control module can temporarily pause the initiation of the "talk to patient" function until the patient has completed the sentence and then continue with pushing the button and transmitting the remote expert's voice (although with a larger initial lag/delay in this case).

Once the audio and button control module 510 receives a signal from the speech detection module that the speech has ended, it instructs the button pusher to release the "push to talk" button and instructs the receive audio buffer module (510) to stop playing or passing through the audio signal. After detection of the voice, the robotic button pusher 112 is triggered. The time it takes from start of voice to initiation of intercom connection is the time that the playback will go back in time in the buffer when starting the audio output.

The playback speed may be chosen such that it (a) is sufficient to reach real-time synchronization during the typical time range of talking (several seconds) and (b) is not too much accelerated so that the listener still understands the words and the voice does not sound unnatural. A typical speed increase may be on the order of 10%, assuming that the delay to be bridged by the buffer is 0.5 seconds and the total time of talking is >5 seconds.

One means of performing the acceleration of the voice is to transform the digital audio to Fourier space, shifting to lower frequencies, back-transformation, higher-speed playback.

The two playback parameters (time to go back in time and playback acceleration factor) may also be adapted based on user feedback.

The local operator may hear the playback from the loudspeaker placed near the intercom device. The local operator could, for example, give immediate feedback about the playback speed being ok or too fast by pushing a button on the user interface of the teleradiology system.

The subject could give feedback after the exam regarding voice of the remote operator clipped or not (buffer playback start time assumed correctly?) and voice of the operator unnatural (playback too fast).
The system could then adapt (increase/decrease) the time to go back in the buffer at start of playback and the playback speed according to the (accumulated) feedback.

The detection of speech signal can be realized by a simple threshold on the magnitude of the audio signal received or by an AI-based detection of human speech in the received signal either in the temporal or in the frequency domain. These two methods of detecting the speech signal can be described as follows:
Method A: Average audio signal strength (or power) over a short period of time (~100ms) can be compared with a threshold value. If the signal strength is above the threshold, it is assumed that the operator has started talking.
Method B: Audio signal pattern in Fourier space is compared with a typical frequency pattern of a human voice by pattern matching (simple overlap integral in frequency domain and threshold value). Again, if the pattern is matched, it is assumed that the operator has started talking.

The remote operator audio stream 384 may be fed into an optional buffer 510 to provide the buffered remote operator audio stream 396 to the interface speaker 118. The receive audio buffer module 510 may be configured to preserve remote tech's speech during the time lag between detection of speech at the speech detection module and the robotic button pusher switches on intercom device, this module temporarily records the ongoing speech. The receive audio buffer is preferentially implemented as a ring buffer, so that data can be recorded continuously. One of the advantages of such approach is that the constant buffering enables the remote operator audio to be buffered without preparation.

The receive audio buffer 510 receives control commands from the audio and button control module, instructing it to play the recorded audio signals with a specified temporal delay. The receive audio buffer may also contain functionality to change the speed of the output audio signal without changing its pitch. In at least some embodiments, this can be achieved by transforming the temporally compressed audio signal into the frequency domain via a Fast Fourier Transform (FFT) and then shifting the signal in the frequency domain in such a way that after back-transform to the temporal domain the original pitch is reproduced. Other transformation techniques like Discrete Fourier Transform (DFT) can be used, and FFT is merely an example. It can also be achieved of complemented by leaving out periods of silence from the output audio stream. Using this functionality, the output audio signal can be played in a slightly accelerated speed until the buffer is played out fully and there is no time lag any more between the buffer output and the speech input. Once this situation has been reached, the original input signal can be passed through directly to the output.

The subject audio stream 390 is received from the interface microphone 116 and fed into the speech and alarm detection module 501. This module is capable of detecting patient speech and alarm signal from the scanner room. The speech detection can be based on audio level detection and AI-based speech recognition, analogous to the speech detection module for the incoming remote audio described above. The speech and alarm signal detection module are further capable of detecting the patient alarm / emergency bell or any other sound of interest that might affect the clinical workflow in unexpected ways. The alarm signal detection can be implemented by pattern recognition in the frequency space or by AI-based temporal signal detection.

If there is an alarm or speech detected by the speech and alarm detection module 501 this goes to the audio and button control module 502. This for example may be able to provide the control signal 388 to activate the robotic button pusher 112. The audio and button control module 502 determines the time when the "talk to patient" function becomes active (or the time delay between initiation of final activation of the talk function) either by receiving a corresponding acknowledgement from the Robotic button pusher 112, or by being provided with a pre-defined delay configuration setting specific for the type of button pusher and the model of the intercom device.

When an alarm signal is communicated to the audio and button control module 502, it can further forward this information to the network interface, so that the remote operator can be notified by the workstation audio or video equipment in an acoustic or optical way about the alarm event.

Once the "talk to patient" function is activated, the audio and button control module instructs the receive audio buffer module 510 to start playing the recorded audio buffer with a negative time offset corresponding to the time it took to activate the "talk to patient" function. It may optionally also instruct the receive audio buffer module to accelerate the output audio (without changing pitch) until the delay between input and output audio has decreased to (almost) zero. Once the read pointer of the buffer has reached the write pointer (due to increased playback speed), the playback speed will be set to real time, thereby simply passing through the buffer input to the buffer output until the voice of the remote operator is not detected any more.

Once the audio and button control module 502 receives a signal from the speech detection module that the speech has ended, it instructs the button pusher to release the "push to talk" button and instructs the receive audio buffer module to stop playing or passing through the audio signal.

In one example, the robotic button pusher 112 is configured to work with a touchscreen button instead of a physical button through attachment of a soft and touch-screen-active material on the top of the mechanical arm. The speech detection module 382 is also able to cause the audio and button control module 502 to generate the control signal 388. There are additionally delay configuration settings 504 which may be used to delay the audio and button control module 502 as well as control the operation of the buffer 510.

The delay configuration settings 504 is a module that contains pre-defined delay times, specifying the expected delay between a speech detection event and a fully established "talk to patient" mode after mechanical interaction with the "push to talk" button. The delay time may depend on the imaging system type and manufacturer, the intercom type and manufacturer, and the technical setup of the robotic button pusher. Multiple delay times may be pre-configured, while only the one valid for the current configuration is used.

In an example, when a remote operator starts talking, the voice is received by the microphone 508, and then transmitted through network interface 372 to speech detection module 382. This module is capable of detecting a refined voice signal (with background noise filtered). As soon as it is certain that the operator is talking (in this example, 0.5 seconds after start of the speech), the audio and button control module 502 initiates the mechanical pushing of the "talk to patient" (push-to-talk) button 102, an operation that takes another 1.1 seconds to complete. The receive audio buffer starts playing the recorded voice of the remote operator, starting at a total offset of -1.6 seconds. The operator is talking for 10 seconds. During the first 5 seconds, the receive audio buffer plays the recorded audio with a slightly increased speed, so that after 5 seconds there is no significant delay between input and output audio anymore. When the remote operator stops talking, the audio detection module will recognize a silence period and the audio and button control module will instruct the button pusher to release the button.

In one example, the talk function of the intercom is switched on and off via a software interface instead of a hardware button. In this case, the robotic button pusher is replaced with a software API connection. Delay configuration settings may be adapted accordingly to consider the delays induced by the software-operated audio direction changes.

In at least some examples, a two-way communication system comprising a remote communication node with a first audio detection device is disclosed. The remote communication node (e.g., a microphone of a remote expert, a reception device) and a local communication node with a second audio detection device (e.g., patient, possibly a local expert) is used wherein the first and second audio detection devices are configured to detect a surrounding sound (e.g., voice). Based on the detected sound transmission, the sound is toggled or prioritized or otherwise stressed, wherein the detected sound is further enhanced for preventing unwanted noise signals or audio clippings.

In some examples, the robotic button pusher is utilized for toggling or prioritizing the detected sound transmission, wherein the robotic button pusher is configured to receive and acknowledge the incoming signals from the first and/or second audio detection devices or an audio control module and determine the two-way communication parameters.

In some examples, the system further comprises a voice pattern determination media, wherein the surrounding sound or voice from a second audio detection devices are analyzed, and if the voice deviates from pre-determined patterns, a notification, such as an alarm, is transmitted to a remote communication node.

In some examples, the detected surrounding sound or voice is further improved algorithmically to improve the quality of detected sound.

In some embodiments, the system further can be configured through delay communication settings, such as having a time-lag from the device.

In some examples, a control message can be utilized. As an example, when human speech is detected, the module informs the `Audio and button control module'. In addition to signaling the detection, the control message also contains a time stamp of the time when the speech started. In some cases, it may take some time (e.g., one second) before the speech recognition system is certain about an ongoing spoken sentence. The time stamp of when the speech started will then point to one second in the past.

It is understood that one or more of the aforementioned examples or embodiments of the invention may be combined as long as the combined embodiments are not mutually exclusive.

As will be appreciated by one skilled in the art, aspects of the present invention may be embodied as an apparatus, method or computer program product. Accordingly, aspects of the present invention may take the form of an entirely hardware embodiment, an entirely software embodiment (including firmware, resident software, micro-code, etc.) or an embodiment combining software and hardware aspects that may all generally be referred to herein as a "circuit," "module" or "system." Furthermore, aspects of the present invention may take the form of a computer program product embodied in one or more computer readable medium(s) having computer executable code embodied thereon.

Any combination of one or more computer readable medium(s) may be utilized. The computer readable medium may be a computer readable signal medium or a computer readable storage medium. A 'computer-readable storage medium' as used herein encompasses any tangible storage medium which may store instructions which are executable by a processor or computational system of a computing device. The computer-readable storage medium may be referred to as a computer-readable non-transitory storage medium. The computer-readable storage medium may also be referred to as a tangible computer readable medium. In some embodiments, a computer-readable storage medium may also be able to store data which is able to be accessed by the computational system of the computing device. Examples of computer-readable storage media include, but are not limited to: a floppy disk, a magnetic hard disk drive, a solid state hard disk, flash memory, a USB thumb drive, Random Access Memory (RAM), Read Only Memory (ROM), an optical disk, a magneto-optical disk, and the register file of the computational system. Examples of optical disks include Compact Disks (CD) and Digital Versatile Disks (DVD), for example CD-ROM, CD-RW, CD-R, DVD-ROM, DVD-RW, or DVD-R disks. The term computer readable-storage medium also refers to various types of recording media capable of being accessed by the computer device via a network or communication link. For example, data may be retrieved over a modem, over the internet, or over a local area network. Computer executable code embodied on a computer readable medium may be transmitted using any appropriate medium, including but not limited to wireless, wire line, optical fiber cable, RF, etc., or any suitable combination of the foregoing.

A computer readable signal medium may include a propagated data signal with computer executable code embodied therein, for example, in baseband or as part of a carrier wave. Such a propagated signal may take any of a variety of forms, including, but not limited to, electro-magnetic, optical, or any suitable combination thereof. A computer readable signal medium may be any computer readable medium that is not a computer readable storage medium and that can communicate, propagate, or transport a program for use by or in connection with an instruction execution system, apparatus, or device.

'Computer memory' or 'memory' is an example of a computer-readable storage medium. Computer memory is any memory which is directly accessible to a computational system. 'Computer storage' or 'storage' is a further example of a computer-readable storage medium. Computer storage is any non-volatile computer-readable storage medium. In some embodiments computer storage may also be computer memory or vice versa.

A 'computational system' as used herein encompasses an electronic component which is able to execute a program or machine executable instruction or computer executable code. References to the computational system comprising the example of "a computational system" should be interpreted as possibly containing more than one computational system or processing core. The computational system may for instance be a multi-core processor. A computational system may also refer to a collection of computational systems within a single computer system or distributed amongst multiple computer systems. The term computational system should also be interpreted to possibly refer to a collection or network of computing devices each comprising a processor or computational systems. The machine executable code or instructions may be executed by multiple computational systems or processors that may be within the same computing device or which may even be distributed across multiple computing devices.

Machine executable instructions or computer executable code may comprise instructions or a program which causes a processor or other computational system to perform an aspect of the present invention. Computer executable code for carrying out operations for aspects of the present invention may be written in any combination of one or more programming languages, including an object-oriented programming language such as Java, Smalltalk, C++ or the like and conventional procedural programming languages, such as the "C" programming language or similar programming languages and compiled into machine executable instructions. In some instances, the computer executable code may be in the form of a high-level language or in a pre-compiled form and be used in conjunction with an interpreter which generates the machine executable instructions on the fly. In other instances, the machine executable instructions or computer executable code may be in the form of programming for programmable logic gate arrays.

The computer executable code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present invention are described with reference to flowchart illustrations and/or block diagrams of methods, apparatus (systems) and computer program products according to embodiments of the invention. It is understood that each block or a portion of the blocks of the flowchart, illustrations, and/or block diagrams, can be implemented by computer program instructions in form of computer executable code when applicable. It is further under stood that, when not mutually exclusive, combinations of blocks in different flowcharts, illustrations, and/or block diagrams may be combined. These computer program instructions may be provided to a computational system of a general-purpose computer, special purpose computer, or other programmable data processing apparatus to produce a machine, such that the instructions, which execute via the computational system of the computer or other programmable data processing apparatus, create means for implementing the functions/acts specified in the flowchart and/or block diagram block or blocks.

These machine executable instructions or computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The machine executable instructions or computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

A 'user interface' as used herein is an interface which allows a user or operator to interact with a computer or computer system. A 'user interface' may also be referred to as a 'human interface device.' A user interface may provide information or data to the operator and/or receive information or data from the operator. A user interface may enable input from an operator to be received by the computer and may provide output to the user from the computer. In other words, the user interface may allow an operator to control or manipulate a computer and the interface may allow the computer to indicate the effects of the operator's control or manipulation. The display of data or information on a display or a graphical user interface is an example of providing information to an operator. The receiving of data through a keyboard, mouse, trackball, touchpad, pointing stick, graphics tablet, joystick, gamepad, webcam, headset, pedals, wired glove, remote control, and accelerometer are all examples of user interface components which enable the receiving of information or data from an operator.

A 'hardware interface' as used herein encompasses an interface which enables the computational system of a computer system to interact with and/or control an external computing device and/or apparatus. A hardware interface may allow a computational system to send control signals or instructions to an external computing device and/or apparatus. A hardware interface may also enable a computational system to exchange data with an external computing device and/or apparatus. Examples of a hardware interface include but are not limited to: a universal serial bus, IEEE 1394 port, parallel port, IEEE 1284 port, serial port, RS-232 port, IEEE-488 port, Bluetooth connection, Wireless local area network connection, TCP/IP connection, Ethernet connection, control voltage interface, MIDI interface, analog input interface, and digital input interface.

A 'display' or 'display device' as used herein encompasses an output device or a user interface adapted for displaying images or data. A display may output visual, audio, and or tactile data. Examples of a display include, but are not limited to: a computer monitor, a television screen, a touch screen, tactile electronic display, Braille screen,
Cathode ray tube (CRT), Storage tube, Bi-stable display, Electronic paper, Vector display, Flat panel display, Vacuum fluorescent display (VF), Light-emitting diode (LED) displays, Electroluminescent display (ELD), Plasma display panels (PDP), Liquid crystal display (LCD), Organic light-emitting diode displays (OLED), a projector, and Head-mounted display.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

### REFERENCE SIGNS LIST

- 100: intercom panel
- 102: push-to-talk button
- 104: intercom panel microphone
- 106: intercom panel speaker
- 108: intercom panel interface
- 110: panel hardware interface
- 112: robotic button pusher
- 114: audio interface
- 116: interface microphone
- 118: interface speaker
- 120: data connection
- 200: manual talk button
- 300: medical system
- 302: magnetic resonance imaging system
- 304: magnet
- 306: bore of magnet
- 308: imaging zone
- 309: field of view
- 310: magnetic field gradient coils
- 312: magnetic field gradient coil power supply
- 314: radio-frequency coil
- 316: transceiver
- 318: subject
- 320: subject support
- 330: control room computer
- 332: control room computational system
- 334: control room hardware interface
- 336: control room user interface
- 338: control room memory
- 340: control room machine executable instructions
- 342: pulse sequence commands
- 344: k-space data
- 346: magnetic resonance image
- 350: intercom system
- 352: subject interface
- 354: subject microphone
- 356: subject headphones
- 358: remote user interface
- 360: local computer
- 362: local computational system
- 364: local hardware interface
- 366: network interface
- 368: local memory
- 372: network connection
- 374: remote operator station
- 380: local machine executable instructions
- 382: speech detection module
- 384: remote operator audio stream
- 386: detection of human speech in remote operator audio stream
- 388: control signal
- 390: subject audio stream
- 392: audio noise filtering module
- 394: alarm identification module
- 396: buffered remote operator audio stream
- 398: audio-time compression algorithm
- 400: receive the remote operator audio stream via the network interface
- 402: detect the human speech within the remote operator audio stream by inputting the remote operator stream into the speech detection module as the input digital audio stream
- 404: provide the control signal and the remote operator audio stream to the panel hardware interface when the human speech is detected
- 406: transmit the subject audio stream to the remote operator station
- 500: medical system
- 501: speech and alarm signal detection module
- 502: audio and button control module
- 504: delay configuration settings
- 506: remote workstation
- 508: speaker and microphone or headset

## Claims

1. An intercom panel interface (108) configured for mounting to an intercom panel (100) of a medical imaging system (302), wherein the intercom panel comprises: a push-to-talk (PPT) button (102), an intercom panel microphone (104), and an intercom panel speaker (106), wherein the intercom panel interface further comprises:
- a panel hardware interface (110) configured for communicating with a local computational system (362), wherein the panel hardware interface is configured for receiving a control command from the local computational system, wherein the panel hardware interface is configured for receiving a remote operator audio stream (384) from the local computational system, wherein the panel hardware interface is further configured to transmit a subject audio stream (390) to the local computational system;
- a robotic button pusher (112) configured to actuate the push-to-talk button in response to receiving the control command;
- an interface microphone (116) configured for receiving subject audio from the intercom panel speaker;
- an interface speaker (118) configured for providing remote operator audio to the intercom panel microphone; and
- an audio interface (114) configured for digitizing the subject audio into the subject audio stream, wherein the audio interface is further configured for rendering the remote operator audio stream to the interface speaker.

2. The intercom panel interface of claim 1, wherein the intercom panel interface is configured to cover the push to talk button of the intercom panel such that manual actuation of the push to talk button is prevented, wherein the intercom panel interface further comprises a manual talk button (200), wherein the intercom panel interface is further configured such that pushing the manual talk button generates the control command to actuate the push to talk button of the intercom panel using the robotic button pusher.

3. A medical system (300, 500) comprising,
- the intercom panel interface of claim 1 or 2;
- a local memory (368) storing local machine executable instructions (380) and a speech detection module (382) configured for detecting human speech in an input digital audio stream;
- a local network interface (366) configured to form a network connection with a remote operator station (374);
- the local computational system, wherein execution of the local machine executable instructions causes the local computational system to continually:
- receive (400) the remote operator audio stream via the network interface;
- detect (402) the human speech within the remote operator audio stream by inputting the remote operator stream into the speech detection module as the input digital audio stream;
- provide (404) the control signal and the remote operator audio stream to the panel hardware interface when the human speech is detected; and
- transmit (406) the subject audio stream to the remote operator station.

4. The medical system of claim 2, wherein the speech detection module is configured to detect the human speech using any one of the following:
- by determining when the average audio signal is above a predetermined signal strength for a least a predetermined time;
- comparing an FFT of the digital audio stream against a predetermined FFT distribution;
- using a trained neural network.

5. The medical system of claim 3 or 4, wherein execution of the machine executable instructions further causes the computational system to buffer (510) the remote operator audio stream.

6. The medical system of claim 5, wherein execution of the machine executable instructions further causes the computational system to delay transmission of the remote operator audio stream using the buffered remote operator audio stream (396) by a predetermined robot button pusher delay.

7. The medical system of claim 5 or 6, wherein execution of the machine executable instructions further causes the computational system to continually:
- detect the human speech within the subject audio stream by inputting the subject audio stream into the speech detection module as the digital audio stream;
- delay the transmission of control command when the human speech is detected in the subject audio stream; and
- delay the transmission of the remote operator audio stream when the subject speech is detected in the subject audio stream using the buffered remote operator audio stream.

8. The medical system of claim 6 or 7, wherein execution of the machine executable instructions further causes the computational system to generate a time-compressed remote operator audio stream from the buffered remote operator audio stream when the buffered remote operator audio stream is delayed with respect to the remote operator audio stream, and wherein the time-compressed remote operator audio stream is transmitted to the panel hardware interface as the remote operator audio stream.

9. The medical system of claim 6, 7, or 8, wherein execution of the machine executable instructions further causes the computational system to pause the remote operator audio stream using the buffered remote operator audio stream when the push to talk button is manually pressed or when local operator speech is detected in the subject audio stream.

10. The medical system of any one of claims 3 through 9, wherein the local memory further stores an audio noise filtering module (392) configured for removing audio noise, wherein execution of the machine executable instructions causes the computational system to remove audio noise from any one of the following using the audio noise filtering module: the remote operator audio stream and the subject audio stream.

11. The medical system of any one of claims 3 through 10, wherein the local memory further comprises an alarm identification module (394) configured to output an alarm signal in response to detecting an audible alarm in the subject audio stream, wherein execution of the machine executable instructions further causes the computational system to:
- detect the audible alarm by inputting the subject audio stream into the alarm identification module; and
- provide an alarm signal to the medical system if the audible alarm is detected.

12. The medical system of any one of claims 3 through 11, wherein the medical system further comprises the remote operator station, wherein the remote operator station is configured for remote control of the medical imaging system.

13. The medical system of any one of claims 3 through 12, wherein the medical system further comprises the medical imaging system and/or wherein the medical system further comprises an intercom system, wherein the intercom system comprises the intercom panel.

14. A method of operating a medical system according to any one of claims 3 through 13, wherein the method comprises:
- receiving (400) the remote operator audio stream via the network interface;
- detecting (402) the human speech within the remote operator audio stream by inputting the remote operator stream into the speech detection module as the input digital audio stream;
- providing (404) the control signal and the remote operator audio stream to the panel hardware interface when the human speech is detected; and
- transmitting (406) the subject audio stream to the remote operator station.

15. A computer program comprising machine executable instructions configured for controlling a local computational system to perform the method of claim 14.
